# EUROPEAN PATENT APPLICATION

(11) **EP 4 506 807 A1**
(43) Date of publication of application: **12.02.2025**
(21) Application number: 23784613.4
(22) Date of filing: 17.03.2023
(51) Int. Cl.: G06F 3/16, A61B 5/11, A61B 5/256, A61B 5/389, A61B 7/04, G06F 3/01, G06F 3/023, G10L 15/22, H04R 1/00

(54) **INFORMATION CONVERSION SYSTEM, INFORMATION PROCESSING DEVICE, INFORMATION PROCESSING METHOD, AND PROGRAM**

(30) Priority: 08.04.2022 JP 2022064525
(71) Applicant: CANON KABUSHIKI KAISHA, Tokyo 146-8501 (JP)
(72) Inventor: MUKAIDE Taihei, Tokyo 146-8501 (JP); KONDOH Takeshi, Tokyo 146-8501 (JP); MASHIDA Ayano, Tokyo 146-8501 (JP)
(74) Representative: Canon Europe Limited
(86) International application number: PCT/JP2023/010507
(87) International publication number: WO 2023/195323

(57) **Abstract**

There is provided an information conversion system capable of converting biometric information into text information or audio information, converting sound information into text information, and outputting highly-accurately-converted text information or audio information. The information conversion system includes: a biometric information detection unit 102 that detects biometric information from one or more parts of a user; a sound information detection unit 103 that detects sound information; a determination unit 110 that determines whether a characteristic of the sound information satisfies a certain condition; and a conversion unit 106 that outputs text information or audio information converted from the biometric information detected by the biometric information detection unit 102 using a first conversion method in a case where the characteristic of the sound information detected by the sound information detection unit 103 does not satisfy the certain condition, and outputs text information converted from the sound information detected by the sound information detection unit 103 using a second conversion method in a case where the characteristic of the sound information satisfies the certain condition.

## Description

### Technical Field

The present invention relates to an information conversion system, an information processing apparatus, an information processing method, and a program for converting biometric information into text information.

### Background Art

In recent years, it has been performed to recognize the user's speech content using the user's audio information. Biometric information indicating the user's mouth movement as well as audio information are obtained, and the recognition results of speech content based on the audio information are output (for example. see PTL 1).

### Citation List

### Patent Literature

PTL 1: Japanese Patent Laid-Open No. 2021-81527

### Summary of Invention

### Technical Problem

However, the configuration in PTL 1 merely outputs the recognition results of speech content based on speech information including the user's audio information. The configuration converts both biometric information and audio information into text information, rather than converting only biometric information into text information.

It is an object of the present invention to be able to convert biometric information into text information or audio information, to convert sound information into text information, and to output highly-accurately-converted text information or audio information.

Note that the problem to be solved by embodiments disclosed in the present specification and drawings are not limited to the above-mentioned problem. Problems corresponding to the effects of each configuration discussed in the embodiments described below can also be regarded as other problems.

### Solution to Problem

To achieve the object of the present invention, an information conversion system of the present invention includes: a biometric information detection unit that detects biometric information from one or more parts of a user; a sound information detection unit that detects sound information; a determination unit that determines whether a characteristic of the sound information satisfies a certain condition; and a conversion unit that outputs text information or audio information converted from the biometric information detected by the biometric information detection unit using a first conversion method in a case where the characteristic of the sound information detected by the sound information detection unit does not satisfy the certain condition, and outputs text information converted from the sound information detected by the sound information detection unit using a second conversion method in a case where the characteristic of the sound information satisfies the certain condition.

Additionally, an information processing apparatus of the present invention includes: a receiving unit that receives biometric information detected by a biometric information detection unit and sound information detected by a sound information detection unit; a determination unit that determines whether a characteristic of the sound information satisfies a certain condition; and a conversion unit that outputs text information or audio information converted from the biometric information detected by the biometric information detection unit using a first conversion method in a case where the characteristic of the sound information detected by the sound information detection unit does not satisfy the certain condition, and outputs text information converted from the sound information detected by the sound information detection unit using a second conversion method in a case where the characteristic of the sound information satisfies the certain condition.

Furthermore, an information processing method of the present invention includes: a step of receiving biometric information detected by a biometric information detection unit and sound information detected by a sound information detection unit; a step of determining whether a characteristic of the sound information satisfies a certain condition; and a step of outputting text information or audio information converted from the biometric information detected by the biometric information detection unit using a first conversion method in a case where the characteristic of the sound information detected by the sound information detection unit does not satisfy the certain condition, and outputting text information converted from the sound information detected by the sound information detection unit using a second conversion method in a case where the characteristic of the sound information satisfies the certain condition.

### Advantageous Effects of Invention

According to the present invention, biometric information can be converted into text information or audio information, sound information can be converted into text information, and highly-accurately-converted text information or audio information can be output.

### Brief Description of Drawings

[Fig. 1] Fig. 1 is a diagram illustrating the configuration of an information conversion system of the present invention.
[Fig. 2] Fig. 2 is a flowchart illustrating the operation of the information conversion system of the present invention.
[Fig. 3] Fig. 3 is a diagram illustrating the configuration of an information conversion system of a first embodiment of the present invention.
[Fig. 4A] Fig. 4A is a schematic diagram of a detection device of the first embodiment of the present invention.
[Fig. 4B] Fig. 4B is a schematic diagram of the detection device of the first embodiment of the present invention.
[Fig. 5] Fig. 5 is a flowchart illustrating an operation related to the first embodiment of the present invention.
[Fig. 6] Fig. 6 is a diagram illustrating the configuration of an information conversion system of a second embodiment of the present invention.
[Fig. 7A] Fig. 7A is a schematic diagram of a detection device of the second embodiment of the present invention.
[Fig. 7B] Fig. 7B is a schematic diagram of the detection device of the second embodiment of the present invention.
[Fig. 8] Fig. 8 is a flowchart illustrating an operation related to the second embodiment of the present invention.
[Fig. 9A] Fig. 9A is a schematic diagram of a detection device of a third embodiment of the present invention.
[Fig. 9B] Fig. 9B is a schematic diagram of the detection device of the third embodiment of the present invention.
[Fig. 10] Fig. 10 is a diagram illustrating the configuration of an information conversion system of a fourth embodiment of the present invention.
[Fig. 11A] Fig. 11A is a schematic diagram of a detection device of the fourth embodiment of the present invention.
[Fig. 11B] Fig. 11B is a schematic diagram of the detection device of the fourth embodiment of the present invention.
[Fig. 12] Fig. 12 is a flowchart illustrating an operation related to the fourth embodiment of the present invention.
[Fig. 13] Fig. 13 is a diagram illustrating the configuration of an information conversion system of a fifth embodiment of the present invention.
[Fig. 14A] Fig. 14A is a schematic diagram of a detection device of the fifth embodiment of the present invention.
[Fig. 14B] Fig. 14B is a schematic diagram of the detection device of the fifth embodiment of the present invention.
[Fig. 15] Fig. 15 is a flowchart illustrating an operation related to the fifth embodiment of the present invention.
[Fig. 16A] Fig. 16A is a schematic diagram of a detection device of a sixth embodiment of the present invention.
[Fig. 16B] Fig. 16B is a schematic diagram of the detection device of the sixth embodiment of the present invention.

### Description of Embodiments

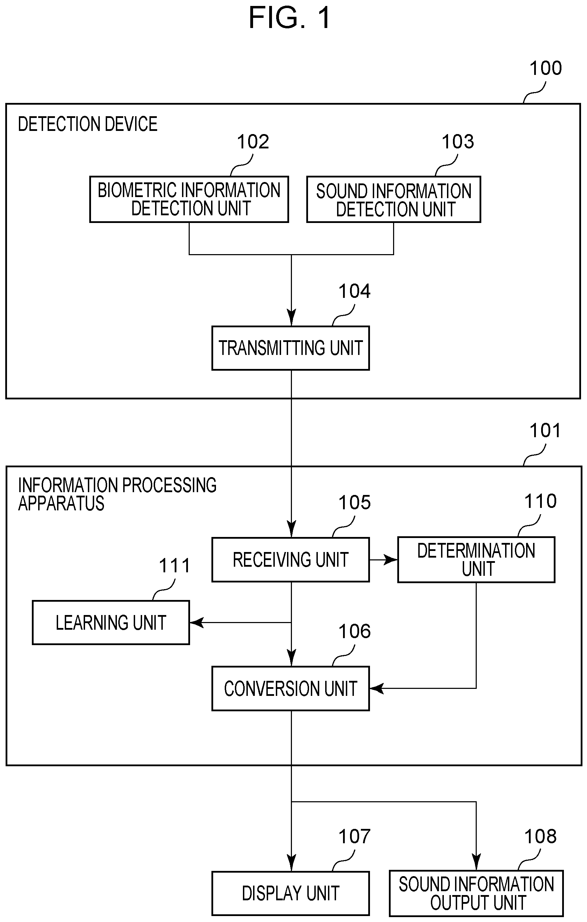

Hereinafter, embodiments of the present invention will be described in detail. Fig. 1 illustrates the outline of an information conversion system of the present invention.

The information conversion system is mainly configured by a detection device 100 and an information processing apparatus 101. The detection device 100 includes a biometric information detection unit 102 that detects biometric information from one or more parts of a user, a sound information detection unit 103 that detects sound information, and a transmitting unit 104 that transmits biometric information detected by the biometric information detection unit 102 and sound information detected by the sound information detection unit 103 to the information processing apparatus 101.

Although Fig. 1 illustrates a form in which the detection device 100 is composed of one device, it may be composed of multiple devices. The biometric information detection unit 102 and the sound information detection unit 103 may be configured by respective detection devices. Note that the detection device 100 can also be rephrased as an obtaining unit that obtains information such as biometric information and sound information.

The biometric information detection unit 102 is composed of a sensor that detects biometric information related to the user's muscle movements, skin, tongue movements, and the like. The biometric information detection unit 102 includes at least one of a myoelectric sensor, an acceleration sensor, an ultrasonic sensor, a tactile sensor, a light sensor, a pressure sensor, and the like. Note that the biometric information detection unit 102 may include a sensor other than those mentioned above as long as it is a sensor that detects biometric information. It is desirable for the biometric information detection unit 102 to be placed in a body part such as the neck, lower jaw, around the mouth, temples, etc., in order to detect biometric information related to the user's mouth or tongue movements. The biometric information detection unit 102 may be placed in a location other than the aforementioned body parts, as long as it can detect biometric information related to the mouth or tongue movements.

The sound information detection unit 103 is composed of a sensor (microphone) that detects external environmental sounds, as well as sounds generated by the user, such as sounds during the user's speech and non-speech sounds like coughing, chewing, and swallowing. The sound information detection unit 103 includes at least one of a condenser microphone, a bone conduction microphone, a skin conduction microphone, and the like. The sound information detection unit 103 may include a sensor other than those mentioned above as long as it detects sounds generated by the user. Also, in the sound information detection unit 103, a sensor (microphone) that detects external environmental sounds and a sensor (microphone) that detects sounds generated by the user, such as sounds during the user's speech and non-speech sounds like coughing, chewing, and swallowing, may be placed separately.

The sound information detection unit 103 may be composed of at least two detection units. The sound information detection unit 103 is, for example, a sound information detection unit that detects sound information generated within the body and a sound information detection unit that detects external sounds. The sound information detection unit 103 that detects sound information generated within the body is, for example, a bone conduction microphone or a skin conduction microphone.

The information processing apparatus 101 includes a receiving unit 105 that receives biometric information and sound information transmitted from the detection device 100, a conversion unit 106 that converts the biometric information and sound information received by the receiving unit 105 into text information and audio information or into audio information, and a determination unit 110 that analyzes the sound information received by the receiving unit 105 to determine whether the characteristics of the sound information satisfy a certain condition.

The information processing apparatus 101 indicates devices such as smartphones, personal computers (PCs), and tablet PCs, but is not limited to these. In the case where the information processing apparatus 101 is a personal computer, for example, text information converted by the conversion unit 106 is transmitted to a display unit 107, such as a display. The display unit 107 displays the text information. The information processing apparatus 101 may include a display control unit (not illustrated) that controls a display form of the display unit 107.

The conversion unit 106 can further convert the converted text information into audio information. Alternatively, the conversion unit 106 can directly convert the biometric information into audio information. The audio information converted by the conversion unit 106 is transmitted to a sound information output unit 108. The sound information output unit 108 is a speaker, and the sound information output unit 108 can play the audio information.

The determination unit 110 analyzes the characteristics of sound information received by the receiving unit 105 and determines whether the characteristics of the sound information satisfy a certain condition. The determination unit 110 determines, for example, whether the volume of the sound information received by the receiving unit 105 is greater than a certain threshold. In addition, the determination unit 110 determines whether the sound information contains noise (sound other than speech) based on the characteristics of the sound information received by the receiving unit 105. Furthermore, the determination unit 110 can also determine whether it is a speech period during which the user is speaking (i.e., whether the user's speech is included) based on the characteristics of the sound information received by the receiving unit 105. The user can arbitrarily select the above determination conditions of the determination unit 110. Noise is, for example, information unrelated to speech generated within the user's body.

Note that the user can also select the above determination conditions of the determination unit 110 in any combination. The determination unit 110 can simultaneously determine, for example, whether the volume of the sound information received by the receiving unit 105 is greater than a certain threshold and whether the sound information contains noise.

Additionally, the determination unit 110 can simultaneously determine, for example, whether it is a speech period during which the user is speaking (whether the user's speech is included) and whether the sound information contains noise, based on the characteristics of the sound information received by the receiving unit 105.

In the case where the determination unit 110 determines that the characteristics of the sound information satisfy a certain condition, it is highly likely that the user's spoken audio has been properly detected, and accordingly the conversion unit 106 converts the sound information detected by the sound information detection unit 103 into text information.

For example, in the case where the determination unit 110 determines that the volume of the sound information is greater than a certain threshold, the conversion unit 106 converts the sound information detected by the sound information detection unit 103 into text information.

In addition, in the case where the volume of the user's spoken sound information received by the receiving unit 105 is greater than a certain threshold, and the sound information contains no noise or contains little noise, the conversion unit 106 converts the sound information detected by the sound information detection unit 103 into text information. Note that, in the case where the determination unit 110 determines that the S/N of the sound related to the user's speech in the sound information is greater than a threshold, the conversion unit 106 may convert the sound information detected by the sound information detection unit 103 into text information. In contrast, in the case where the determination unit 110 determines that the characteristics of the sound information do not satisfy a certain condition, it is highly likely that the user's spoken audio has not been properly detected, and accordingly the conversion unit 106 converts biometric information detected by the biometric information detection unit 102 into text information or audio information.

In the case where the determination unit 110 determines that the volume of the sound information is less than or equal to a certain threshold, the conversion unit 106 converts biometric information detected by the biometric information detection unit 102 into text information or audio information.

In addition, in the case where sound information received by the receiving unit 105 contains noise due to external sounds, and if it is not a speech period during which the user is speaking, the conversion unit 106 converts biometric information detected by the biometric information detection unit 102 into text information or audio information. Note that, in the case where sound information received by the receiving unit 105 contains noise generated within the body, such as coughing, chewing, and swallowing sounds, it is highly likely that the biometric information detected by the biometric information detection unit 102 also contains noise. Therefore, the conversion unit 106 can be set to not convert the biometric information detected by the biometric information detection unit 102 into either text information or audio information. Two types of microphones can be used to identify external sounds and noise generated within the body. For example, a regular microphone for capturing external sounds, as well as a bone conduction microphone or a skin conduction microphone for detecting sounds generated within the body, can be used.

When converting biometric information detected by the biometric information detection unit 102 into text information or audio information, the conversion unit 106 converts the biometric information into text information or audio information using a first conversion method (first conversion algorithm). When converting sound information detected by the sound information detection unit 103 into text information, the conversion unit 106 converts the sound information into text information using a second conversion method (second conversion algorithm).

The conversion algorithm in the first conversion method and the conversion algorithm in the second conversion method use a trained model with an architecture configured by a neural network. The information processing apparatus 100 has a storage unit (not illustrated) that stores the trained model. The conversion unit 106 has the function of making inferences using the trained model.

Trained models include those generated using deep learning, such as CNNs (Convolutional Neural Networks) and RNNs (Recurrent Neural Networks). In addition to models derived from CNNs and RNNs, other machine learning techniques may be used, such as support vector machines, logistic regression, and random forests. Rule-based approaches may also be used.

The information processing apparatus 101 generates a trained model to be used in the first conversion method by, for example, learning biometric information detected by the biometric information detection unit 102 and text information or audio information in association with each other. The information processing apparatus 101 generates a trained model to be used in the second conversion method by, for example, learning sound information detected by the sound information detection unit 103 and text information in association with each other.

Specifically, the information processing apparatus 101 preliminarily obtains multiple data sets of biometric information (e.g., the waveforms of myoelectric signals) detected by the biometric information detection unit 102 and text information or audio information (e.g., a, i, u, e, o, or their corresponding sounds that are associated with each other. The biometric information detection unit 102, for example, has multiple electrodes on the surface that contacts the user's skin (contact surface), and it can measure the user's myoelectric signals at the respective electrodes.

In Fig. 1, a learning unit 111 is configured in the information processing apparatus 101. Note that the learning unit 111 may be configured on the cloud. In the case where the learning unit 111 is configured on the cloud, various types of data are transferred via a data transfer unit (not illustrated) in the information processing apparatus 101.

The learning unit 111 generates a trained model to be used in the first conversion method by learning the correspondence between biometric information and text information or audio information from multiple data sets as training data, associating the biometric information with the text information or audio information. In this way, using the trained model trained by learning biometric information and text information or audio information in association with each other, the conversion unit 106 can make inferences about newly input biometric information and output text information or audio information.

Similarly, the learning unit 111 preliminarily obtains multiple data sets of sound information (e.g., the waveform of sound information) detected by the sound information detection unit 103 and text information (e.g., a, i, u, e, and ∘) that are associated with each other. The learning unit 111 generates a trained model to be used in the second conversion method by learning the correspondence between sound information and text information from multiple data sets as training data, associating the characteristics of the sound information with the text information. In this way, using the trained model trained by learning sound information and text information in association with each other, the conversion unit 106 can make inferences about newly input sound information and output text information.

Although the conversion algorithm in the first conversion method and the conversion algorithm in the second conversion method have been described, a third conversion algorithm that converts information including sound information and biometric information into text information or audio information can also be used.

Specifically, the learning unit 111 preliminarily obtains multiple data sets of information including biometric information detected by the biometric information detection unit 102 and sound information detected by the sound information detection unit 103, and text information or audio information that are associated with each other. The learning unit 111 generates a trained model to be used in the third conversion method by learning the correspondence between information including biometric information and the characteristics of sound information, and text information or audio information from multiple data sets as training data, associating the information including biometric information and sound information with the text information or audio information. In this way, using the trained model trained by learning information including biometric information and sound information, and text information in association with each other, the conversion unit 106 can make inferences about newly input information including biometric information and sound information, and output text information or audio information.

The learning unit 111 can update a trained model to be used in the first conversion method by learning the correspondence between text information converted by the second conversion method using the sound information detected by the sound information detection unit 103 and the biometric information obtained by the biometric information detection unit 102 obtained at the same time as the sound information detected by the sound information detection unit 103 as training data, associating the biometric information with the text information or audio information.

Note that the information processing apparatus 101 may be configured on the cloud. The transmitting unit 104 in the detection device 100 transmits biometric information detected by the biometric information detection unit 102 and sound information detected by the sound information detection unit 103 to the cloud. In the cloud, the biometric information and sound information are converted into text information or audio information, and the converted text information is transmitted to the display unit 107. The display unit 107 displays the text information. Additionally, the converted text information may be further converted into audio information and transmitted to the sound information output unit 108. Alternatively, the biometric information may be directly converted into audio information and transmitted to the sound information output unit 108.

It is acceptable for communication between the detection device 100 and the information processing apparatus 101 to be either wired or wireless. In the case where communication between the detection device 100 and the information processing apparatus 101 is realized as wired, the transmitting unit 104 in the detection device 100 and the receiving unit 105 in the information processing apparatus 101 are connected by a cable such as a USB cable or HDMI (registered trademark).

In the case where communication between the detection device 100 and the information processing apparatus 101 is realized as wireless, the transmitting unit 104 in the detection device 100 and the receiving unit 105 in the information processing apparatus 101 are connected wirelessly by wireless LAN communication such as WiFi, or by short-range wireless communication such as Bluetooth (registered trademark).

When the information processing apparatus 101 is a smartphone or tablet PC, the display unit 107 is a display. The sound information output unit 108 can be a speaker installed in the smartphone or tablet PC, or earphones connected to the smartphone or tablet PC.

The information conversion system of the present invention includes: the biometric information detection unit 102, which detects biometric information from one or more parts of a user; the sound information detection unit 103, which detects sound information; the determination unit 110, which determines whether a characteristic of the sound information satisfies a certain condition; and the conversion unit 106, which outputs text information or audio information converted from the biometric information detected by the biometric information detection unit 102 using a first conversion method in a case where the characteristic of the sound information detected by the sound information detection unit 103 does not satisfy the certain condition, and outputs text information converted from the sound information detected by the sound information detection unit 103 using a second conversion method in a case where the characteristic of the sound information satisfies a certain condition.

In the case where the characteristic of the sound information detected by the sound information detection unit 103 does not satisfy the certain condition, the conversion unit 106 can convert the biometric information detected by the biometric information detection unit 102 into text information or audio information using the first conversion method, and, in the case where the characteristic of the sound information satisfies the certain condition, the conversion unit 106 can convert the sound information into text information using the second conversion method.

The conversion unit 106 can switch from the first conversion method to the second conversion method or from the second conversion method to the first conversion method depending on the characteristic of the sound information detected by the sound information detection unit 103, and output text information or audio information converted by the switched conversion method.

In the case where the characteristic of the sound information detected by the sound information detection unit 103 satisfies the certain condition, the conversion unit 106 can also output text information converted from information including the sound information and the biometric information using a third conversion method. The conversion unit 106 can switch from the first conversion method to the third conversion method or from the third conversion method to the first conversion method depending on the characteristic of the sound information detected by the sound information detection unit 103, and output text information or audio information converted by the switched conversion method.

In the case where the determination unit 110 determines that the sound information detected by the sound information detection unit 103 contains noise, the conversion unit 106 can be set to not convert the biometric information into either text information or audio information.

In the case where the determination unit 110 determines that the volume of the sound information detected by the sound information detection unit 103 is greater than a threshold, the conversion unit 106 can convert the sound information into text information; and, in the case where the determination unit 110 determines that the volume of the sound information detected by the sound information detection unit 103 is less than or equal to the threshold, the conversion unit 106 can convert the biometric information into text information.

In the case where the determination unit 110 determines that the sound information detected by the sound information detection unit 103 corresponds to a user's speech period, the conversion unit 106 converts the sound information into text information. In the case where the determination unit 110 determines that the sound information detected by the sound information detection unit 103 corresponds to a user's non-speech period, the conversion unit 106 converts the biometric information into text information or audio information.

Depending on the characteristic of the sound information detected by the sound information detection unit 103, the conversion unit 106 sets either the first conversion method of converting biometric information into text information or audio information or the second conversion method of converting sound information into text information, and converts the biometric information or sound information into text information or audio information. The information processing apparatus 101 of the present invention includes: the receiving unit 105, which receives biometric information detected by the biometric information detection unit 102 and sound information detected by the sound information detection unit 103; the determination unit 110, which determines whether a characteristic of the sound information satisfies a certain condition; and the conversion unit 106, which outputs text information or audio information converted from the biometric information detected by the biometric information detection unit 102 using a first conversion method in a case where the characteristic of the sound information detected by the sound information detection unit 103 does not satisfy the certain condition, and outputs text information converted from the sound information detected by the sound information detection unit 103 using a second conversion method in a case where the characteristic of the sound information satisfies a certain condition. In the case where the characteristic of the sound information detected by the sound information detection unit 103 satisfies a certain condition, the conversion unit 106 can also output text information or audio information converted from information including the sound information and the biometric information using the third conversion method.

Note that the information processing apparatus 101 may also include a processing unit that evaluates the biometric information detected by the biometric information detection unit 102 based on a certain evaluation criterion and deletes text information or audio information corresponding to the biometric information not satisfying the certain evaluation criterion.

Thus, according to the present invention, highly-accurately-converted text information or audio information can be output.

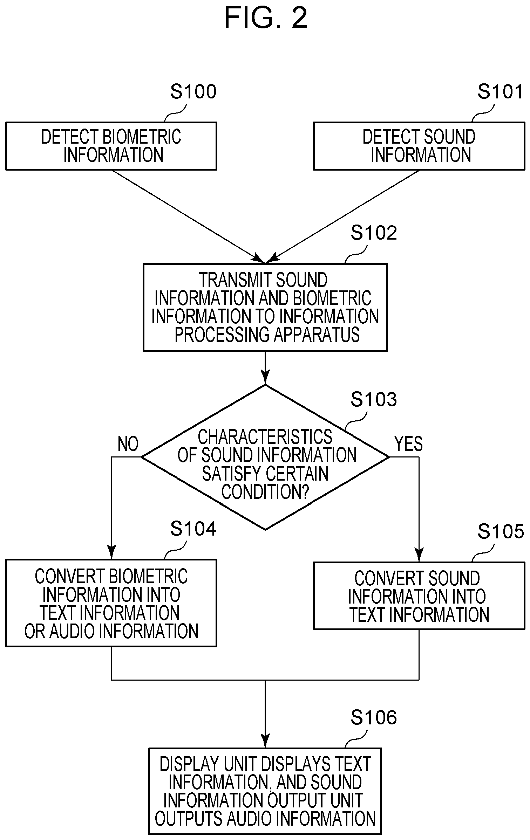

Fig. 2 illustrates a flowchart indicating the operation of the information conversion system of the present invention.

The user wears the detection device 100 at a position where biometric information can be detected by the biometric information detection unit 102. The user wears the detection device 100 on the head, neck, periphery of the head or neck, etc. The biometric information detection unit 102 detects biometric information including at least one of a myoelectric signal, acceleration information, ultrasonic information, tactile information, light information, pressure information, and the like. When the biometric information detection unit 102 detects biometric information, it obtains time information (time of day information). Biometric information is accompanied by auxiliary information related to time information (S100).

The user attaches the detection device 100 to a position where sound information can be detected by the sound information detection unit 103. The user attaches the detection device 100 anywhere from the user's neck to head, at a position where environmental sounds around the user can be detected. The sound information detection unit 103 detects sound information at the same timing as the biometric information detection unit 102 detects biometric information. When the sound information detection unit 103 detects sound information, it obtains time information (time of day information). Sound information is accompanied by auxiliary information related to time information (S101).

The transmitting unit 104 transmits the sound information and biometric information to the information processing apparatus 101. The biometric information and sound information are respectively accompanied by auxiliary information related to time information (time of day information). The biometric information and sound information are linked by the auxiliary information. Since the biometric information and sound information are linked by auxiliary information related to time information (time of day information), the transmitting unit 104 can transmit the biometric information and sound information detected in the same time zone (time of day) to the information processing apparatus 101 (S102).

The sound information and biometric information detected by the detection device 100 are received by the receiving unit 105 in the information processing apparatus 101. The receiving unit 105 transmits the sound information and biometric information to the conversion unit 106. The receiving unit 105 transmits the sound information to the determination unit 110. The determination unit 110 analyzes the characteristics of the sound information received by the receiving unit 105 and determines whether the characteristics of the sound information satisfy a certain condition. For example, the determination unit 110 determines whether the sound information includes the user's speech (S103). In the case where the characteristics of the sound information do not satisfy the certain condition, the process transitions to S104; and, in the case where the characteristics of the sound information satisfy the certain condition, the process transitions to S105.

As a result of the determination by the determination unit 110, if the characteristics of the sound information do not satisfy the certain condition, the conversion unit 106 converts the biometric information into text information or audio information using the first conversion method. The conversion unit 106 may also convert the biometric information into audio information, or, after converting the biometric information into text information, the conversion unit 106 may perform audio synthesis to convert it into audio information. The conversion unit 106 outputs the text information converted from the biometric information (S104).

Note that, in the case where biometric information is detected by the biometric information detection unit 102, the determination unit 110 may sometimes determine that sound information detected by the sound information detection unit 103 at that time includes the user's non-speech sound information (e.g., sublingual sounds or coughing). In that case, the conversion unit 106 may be set not to convert the biometric information into either text information or audio information.

As a result of the determination by the determination unit 110, if the characteristics of the sound information satisfy the certain condition, the conversion unit 106 converts the sound information into text information using the second conversion method (S105).

In the case where sound information detected by the sound information detection unit 103 contains noise, the conversion unit 106 may also be set to not convert the sound information into text information using the second conversion algorithm. In addition, if the S/N of the sound information detected by the sound information detection unit 103 is poor, the conversion unit 106 can also convert information including the sound information and biometric information into text information or audio information using a third conversion algorithm that converts information including sound information and biometric information into text information or audio information. Note that, if the *S*/*N* of the sound information detected by the sound information detection unit 103 is poor, the conversion unit 106 may perform conversion using only the biometric information.

The display unit 107 displays the text information converted by the conversion unit 106, and the sound information output unit outputs audio information further converted from the text information by the conversion unit 106 (S106).

Additionally, the text information converted by the conversion unit 106 can be stored in the storage unit in the information processing apparatus 101. The text information can be transferred over a network and displayed at an external terminal.

Furthermore, by converting text information or audio information into audio information, it is possible to play and record it on an external terminal. The user can also listen to the reproduced audio through earphones. This allows the user to check whether the conversion is correct. The audio information may be further transferred over a network and played and recorded at an external terminal. Additionally, the external terminal can be controlled based on the converted text information. By repeatedly performing this series of processes, continuous communication using biometric information can be performed.

### First Embodiment

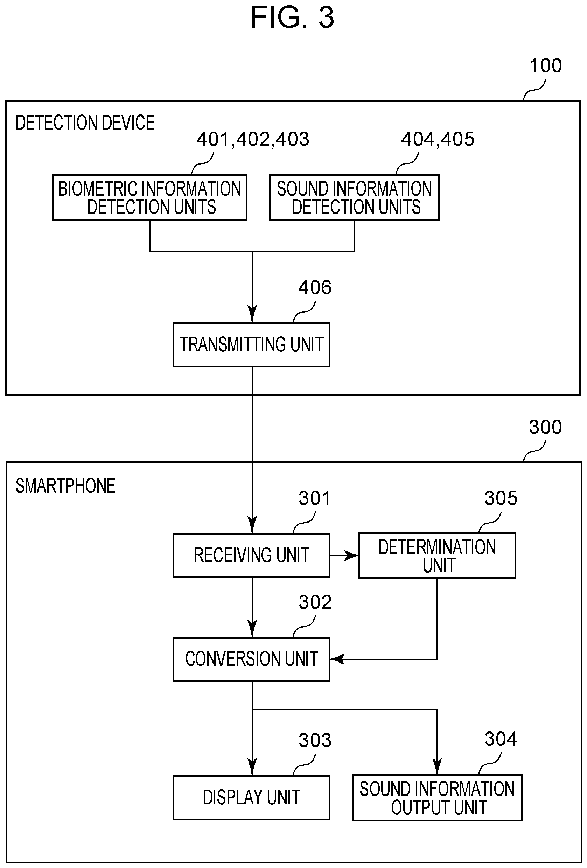

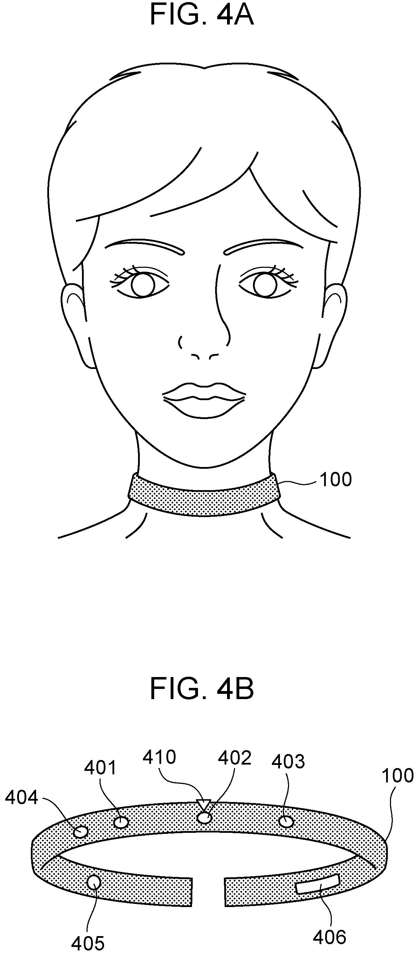

Next, a first embodiment of a text conversion function using the information conversion system of the present invention will be described. Fig. 3 illustrates the outline of the information conversion system of the present invention. Figs. 4A and 4B illustrate schematic diagrams of the detection device 100.

Here, the form in which the information processing apparatus of the information conversion system is a smartphone 300 will be discussed. In the detection device 100, there are multiple biometric information detection units 401, 402, and 403 and sound information detection units 404 and 405. Note that the detection device is supplied with power from a battery (not illustrated). In addition, the smartphone 300 has the same configuration as the information processing apparatus 101 illustrated in Fig. 1 except for the positions of a display unit 303 and a sound information output unit 304, so its description will be omitted.

As illustrated in Fig. 4A, the user wears the detection device 100 on the neck. The detection device 100 is a choker type (annular shape) that is wrapped around and worn on the user's neck, and is made of an elastic member (fabric). The detection device 100 may be provided with a connecting part that connects both ends of the elastic member to be worn on the user's neck. As illustrated in Fig. 4B, the detection device 100 is composed of the biometric information detection units 401, 402, and 403, the sound information detection units 404 and 405, and a transmitting unit 406. The biometric information detection units 401, 402, and 403 and the sound information detection units 404 and 405 are connected to the transmitting unit 406. The transmitting unit 406 can transmit biometric information detected by the biometric information detection units 401, 402, and 403 and sound information detected by the sound information detection units 404 and 405 to the outside.

The biometric information detection units 401, 402, and 403 are, for example, six-axis acceleration sensors. The six-axis acceleration sensors are sensors that can measure, for example, three-axis translational acceleration and three-axis angular acceleration. The biometric information detection units 401, 402, and 403 are installed on the inner side (inner peripheral surface) of the detection device 100, which is a choker type. Therefore, the biometric information detection units 401, 402, and 403 can be brought into contact with the user's skin, and biometric information (various types of acceleration) can be detected by the biometric information detection units 401, 402, and 403. Accordingly, the biometric information detection units 401, 402, and 403 can detect information related to the movement of the user's body part.

The sound information detection unit 404 is, for example, a bone conduction microphone that detects sounds generated within the user. The bone conduction microphone is a device that detects sound information by sensing the vibrations caused by the user's voice transmitted to the user's neck and detecting the vibrations of the neck bones. The sound information detection unit 404 may be a microphone using various conduction methods, such as a skin conduction microphone that detects sound information by sensing the vibrations of the skin on the neck. The sound information detection unit 404 is installed on the inner side (inner peripheral surface) of the detection device 100, which is a choker type. Therefore, the sound information detection unit 404 can be brought into contact with the user's skin and can detect the vibrations of the skin on the neck. Thus, the sound information detection unit 404 can detect sounds generated within the user.

The sound information detection unit 405 is a microphone for capturing external environmental sounds. The sound information detection unit 405 is installed on the outer side (outer peripheral surface) of the detection device 100, which is a choker type. The sound information detection unit 405 can detect external environmental sounds. A marker 410 is a mark for the user to perform alignment. The user can align the center of the neck (such as the throat) with the biometric information detection unit 402 using the marker 410 as an indicator, and the detection device 100 can be suitably worn on the neck.

Biometric information detected by the biometric information detection units 401, 402, and 403 and sound information detected by the sound information detection units 404 and 405 are transmitted by the transmitting unit 406 to the smartphone 300 connected via Bluetooth, for example. A receiving unit 301 in an application on the smartphone 300 receives data. A determination unit 305 analyzes the sound information received by the receiving unit 301 and determines whether the characteristics of the sound information satisfy a certain condition. A conversion unit 302 converts the biometric information or sound information received by the receiving unit 301 into text information or audio information in accordance with the determination result of the determination unit 305. The text information converted by the conversion unit 302 is displayed on a display, which is the display unit 303 of the smartphone. The sound information output unit 304 plays the audio information converted by the conversion unit 302.

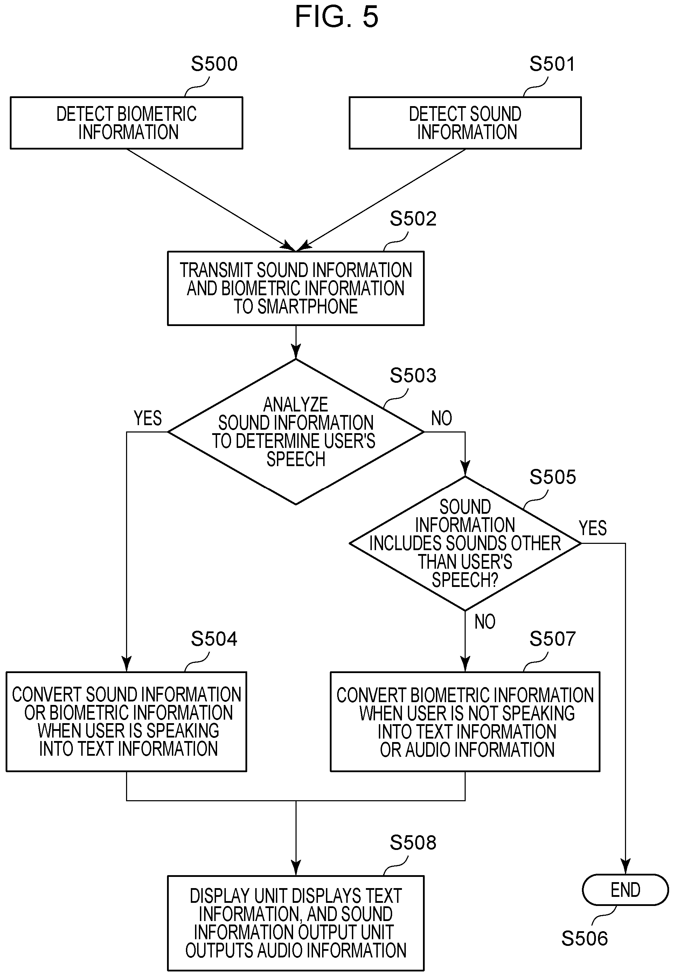

Fig. 5 illustrates a flowchart indicating the operation of the information conversion system of the present invention.

The user wears the detection device 100 on the neck. The user wears the detection device 100 at a position where biometric information can be detected by the biometric information detection units 401, 402, and 403, and sound information can be detected by the sound information detection units 404 and 405. The biometric information detection units 401, 402, and 403 obtain biometric information of the user (S500).

The sampling rate of the biometric information detection units 401, 402, and 403 is set to 1 kHz.

The sound information detection units 404 and 405 detect sound information at the same timing as the biometric information detection units 401, 402, and 403 detect biometric information (S501).

The sampling rate of the sound information detection units 404 and 405 is set to 16 kHz. The transmitting unit 406 transmits the sound information and biometric information to the smartphone 300 (S502).

The sound information and biometric information are transmitted to a conversion unit 306 and the determination unit 305 via the receiving unit 305. The determination unit 305 determines whether the sound information detected by the sound information detection unit 404 includes audio information of the user's speech using a model using a neural network (S503).

In the case where the sound information detected by the sound information detection unit 404 includes audio information of the user's speech, the process proceeds to S504; and, in the case where the sound information detected by the sound information detection unit 404 includes no audio information of the user's speech, the process proceeds to S505.

For each of the case where the sound information detected by the sound information detection unit 404 includes audio information and the case where the sound information includes no audio information, the conversion unit 302 switches the conversion algorithm and converts the sound information or biometric information into text information. The conversion unit 302 converts the sound information or biometric information when the user is speaking into text information or audio information, and outputs the text information (S504).

In the case where the sound information detected by the sound information detection unit 404 includes no audio information, the sound information detection unit 404 obtains sounds related to the user's unintended movements (related to coughing, swallowing saliva, etc.), and the determination unit 305 determines whether the sound information includes sounds other than the user's speech (S505).

In the case where the sound information includes sounds other than the user's speech, the process proceeds to S506; and, in the case where the sound information detected by the sound information detection unit 404 includes no sounds other than the user's speech, the process proceeds to S507.

In the case where the sound information detected by the sound information detection unit 404 includes sounds other than the user's speech, the conversion unit 302 ends the process without performing conversion processing using the information (S506).

In the case where the sound information detected by the sound information detection unit 404 includes no sounds other than the user's speech, the conversion unit 302 converts the biometric information when the user is not speaking into text information or audio information, and outputs the text information (S507).

In the case where the sound information includes no sounds other than the user's speech, the conversion unit 302 converts the biometric information into text information or audio information using the above-mentioned first conversion method. Specifically, the biometric information is converted into text information by using the sensor information from 18 axes, consisting of 6 axes × 3 acceleration sensors, as input data for the first conversion algorithm. Then, the display unit 303 displays the converted text information (S508).

Additionally, even in the case where the sound information detected by the sound information detection unit 404 includes sounds other than the user's speech, if the S/N of the audio information is poor, the biometric information in addition to the audio information may be used to perform conversion. Alternatively, it may be set to perform conversion only from the biometric information.

### Second Embodiment

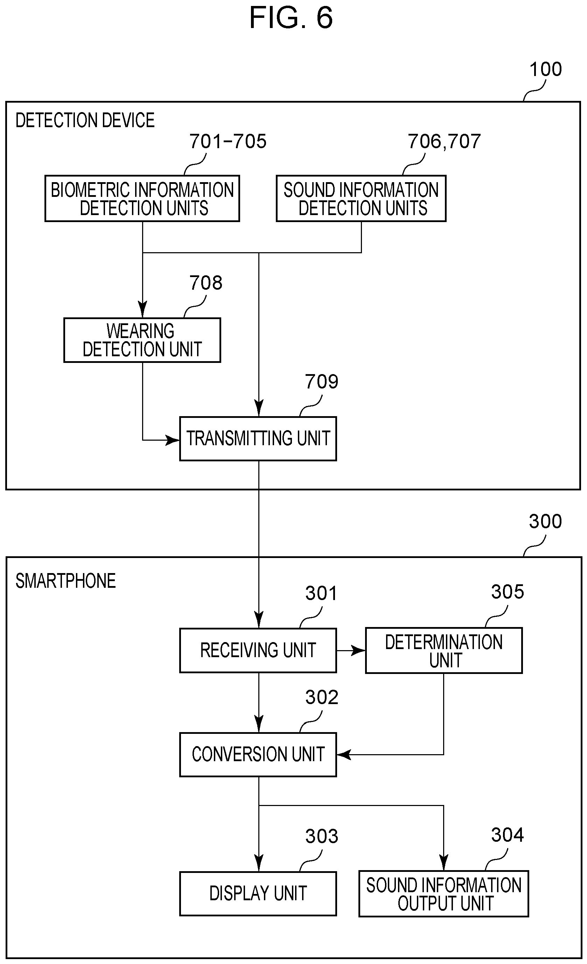

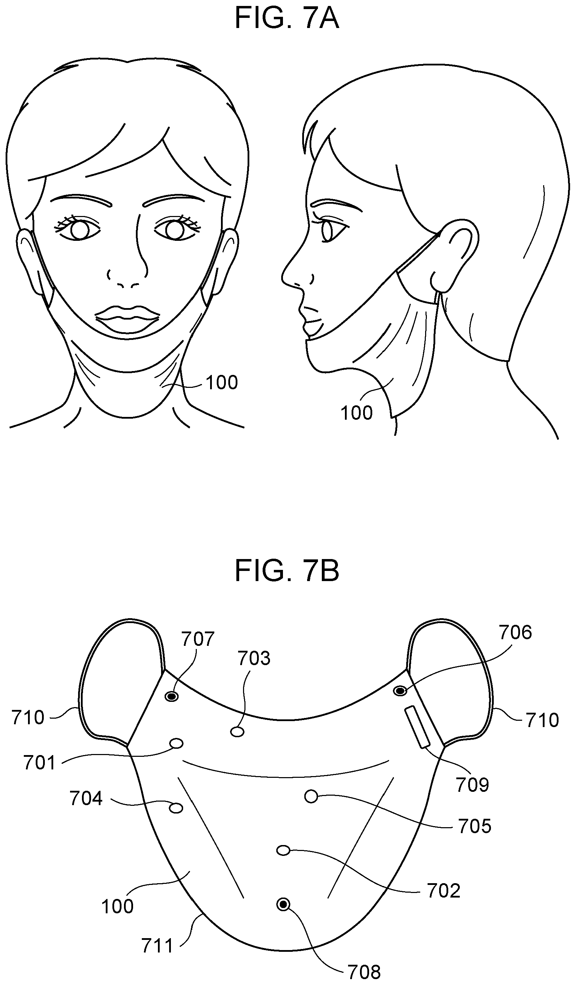

Next, a second embodiment of a text conversion function using the information conversion system of the present invention will be described. Fig. 6 illustrates the outline of the information conversion system of the present invention. Figs. 7A and 7B illustrate schematic diagrams of the detection device 100.

As illustrated in Fig. 6, the detection device 100 has a wearing detection unit 708 that detects that biometric information detection units 701 to 705 have been worn on the user. Because the configuration of the smartphone 300 used as the information processing apparatus is the same as that of the smartphone 300 illustrated in Fig. 3, its description will be omitted here.

In response to detection by the wearing detection unit 708 that the biometric information detection units 701 to 705 have been worn on the user, the conversion unit 302 can convert biometric information detected by the biometric information detection units 701 to 705 into text information. In the case where the wearing detection unit 708 does not detect that the biometric information detection units 701 to 705 have been worn on the user, biometric information detected by the biometric information detection units 701 to 705 cannot be converted into text information or audio information.

The detection device 100 has the biometric information detection units 701 to 705, sound information detection units 706 and 707, and a transmitting unit 709 that transmits biometric information detected by the biometric information detection units and sound information detected by the sound information detection units. The smartphone 300 (information processing apparatus) includes the receiving unit 301, which receives biometric information and audio information transmitted from the transmitting unit 709, the determination unit 305, and the conversion unit 302.

In response to detection by the wearing detection unit 708 that the biometric information detection units 701 to 705 have been worn on the user, the detection device 100 and the information processing apparatus 300 enter a communicable state.

As illustrated in Fig. 7A, the user wears the detection device 100 on the jaw. As illustrated in Fig. 7B, the detection device 100 includes the biometric information detection units 701 to 705, the sound information detection units 706 and 707, the wearing detection unit 708, and the transmitting unit 709.

The detection device 100 is a jaw mask type that is worn on the user's jaw, and is made of an elastic member (fabric). The detection device 100 is formed of two ear loops 710, and a neck-and-jaw contact part 711. The neck-and-jaw contact part 711 is made of an elastic member (fabric).

In the neck-and-jaw contact part 711, the biometric information detection units 701 to 705, the sound information detection units 706 and 707, and the wearing detection unit 708 are arranged. When the user places the ear loops 710 over both ears and wears the detection device 100 on the user, the biometric information detection units 701 to 705, the sound information detection units 706 and 707, and the wearing detection unit 708 are respectively placed on the user's neck and jaw.

The biometric information detection units 701 to 705, the sound information detection units 706 and 707, and the wearing detection unit 708 are connected to the transmitting unit 709. The transmitting unit 709 can transmit biometric information detected by the biometric information detection units 701 to 705, sound information detected by the sound information detection units 706 and 707, and the wearing detection result detected by the wearing detection unit 708 to the outside.

The biometric information detection units 701 to 705 are, for example, myoelectric sensors, and are placed on the neck and lower jaw. The biometric information detection units 701 to 705 are installed on the back surface (contact surface) of the detection device 100. Therefore, the biometric information detection units 701 to 705 can be brought into contact with the user's skin, and the biometric information detection units 701 to 705 can detect biometric information (myoelectric information).

The sound information detection unit 706 is, for example, a bone conduction microphone for detecting sounds generated within the user. The sound information detection unit 706 may be a skin conduction microphone. The sound information detection unit 706 is installed on the back surface (contact surface) of the detection device 100. Therefore, the sound information detection unit 706 can be brought into contact with the user's skin and can detect sounds generated within the user. The sound information detection unit 707 is a microphone for capturing external environmental sounds. The sound information detection unit 707 is installed on the front surface (non-contact surface) of the detection device 100. The sound information detection unit 707 can detect external environmental sounds.

The wearing detection unit 708 monitors the outputs from the myoelectric sensors and detects that the user has worn the detection device 100 on the neck and lower jar. The wearing detection unit 708 detects that the detection device 100 has been worn based on the characteristics of the output waveforms from the myoelectric sensors. The characteristics of the output waveforms from the myoelectric sensors include, for example, the maximum myoelectric potential and the waveform pattern of the myoelectric potential. If the maximum myoelectric potential exceeds a certain threshold, it is detected that the detection device 100 has been worn.

Additionally, if the waveform pattern of the myoelectric potential is a pattern similar to a certain waveform pattern, it is detected that the detection device 100 has been worn.

As illustrated in Fig. **6****,** biometric information detected by the biometric information detection units 701 to 705, sound information detected by the sound information detection units 706 and 707, and the wearing detection result detected by the wearing detection unit 708 are transmitted by the transmitting unit 709 to the smartphone 300 connected via WiFi, for example. The receiving unit 301 in the application on the smartphone 300 receives data. The determination unit 305 analyzes the sound information received by the receiving unit 301 and determines whether the characteristics of the sound information satisfy a certain condition. The determination unit 305 may make a determination using the wearing detection result detected by the wearing detection unit 708.

The conversion unit 302 converts the biometric information or sound information received by the receiving unit 301 into text information in accordance with the determination result of the determination unit 305. The text information converted by the conversion unit 302 is displayed on a display, which is the display unit 303 of the smartphone. The sound information output unit 304 plays the audio information converted by the conversion unit 302.

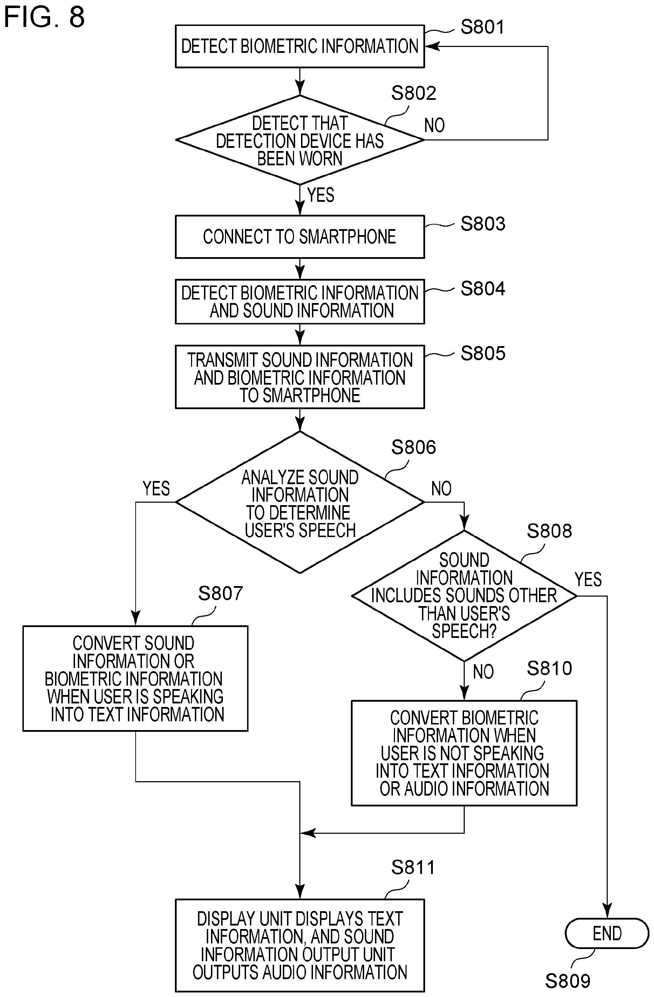

Fig. 8 illustrates a flowchart indicating the operation of the information conversion system of the present invention.

The user wears the detection device 100 at a position where biometric information can be detected by the biometric information detection units 701 to 705, and sound information can be detected by the sound information detection units 706 and 707. After the detection device 100 is powered on, the biometric information detection units 701 to 705 detect biometric information (S801).

The sampling rate of the biometric information detection units 701 to 705 is 2 kHz.

Based on the characteristics of the biometric information (characteristics of the output waveforms from the myoelectric sensors), the wearing detection unit 708 detects that the detection device 100 has been worn (S802).

In response to detection by the wearing detection unit 708 that the detection device 100 has been worn, the detection device 100 and the information processing apparatus 300 enter a communicable state. The transmitting unit 709 in the detection device 100 is connected to the smartphone 300 to be able to transmit various types of information (S803).

The biometric information detection units 701 to 705 obtain biometric information, and the sound information detection units 706 and 707 obtain sound information (S804). The sampling rate of the sound information detection units 706 and 707 has been set to 16 kHz. The transmitting unit 709 transmits the sound information and biometric information to the smartphone 300 (S805).

The sound information and biometric information are transmitted to the conversion unit 306 and the determination unit 305 via the receiving unit 305. The determination unit 305 determines whether the sound information detected by the sound information detection unit 706 includes audio information of the user's speech using a model using a neural network (S806).

In the case where the sound information detected by the sound information detection unit 706 includes audio information of the user's speech, the process proceeds to S807; and, in the case where the sound information detected by the sound information detection unit 706 includes no audio information of the user's speech, the process proceeds to S808.

For each of the case where the sound information detected by the sound information detection unit 706 includes audio information and the case where the sound information includes no audio information, the conversion unit 302 switches the conversion algorithm and converts the sound information or biometric information into text information. The conversion unit 302 converts the sound information or biometric information when the user is speaking into text information, and outputs the text information (S807).

In the case where the sound information detected by the sound information detection unit 706 includes no audio information, the sound information detection unit 706 obtains sounds related to the user's unintended movements (related to coughing, swallowing saliva, etc.), and the determination unit 305 determines whether the sound information includes sounds other than the user's speech (S808).

In the case where the sound information detected by the sound information detection unit 706 includes sounds other than the user's speech, the process proceeds to S809; and, in the case where the sound information detected by the sound information detection unit 706 includes no sounds other than the user's speech, the process proceeds to S810.

In the case where the sound information detected by the sound information detection unit 706 includes sounds other than the user's speech, the conversion unit 302 ends the process without performing conversion processing using that information (S809).

In the case where the sound information detected by the sound information detection unit 706 includes no sounds other than the user's speech, the conversion unit 302 converts the biometric information when the user is not speaking into text information or audio information, and outputs the text information or audio information (S810).

In the case where the sound information detected by the sound information detection unit 706 includes no sounds other than the user's speech, the conversion unit 302 converts the biometric information into text information or audio information using the above-mentioned first conversion method. Specifically, the biometric information is converted into text information or audio information by using the myoelectric information from the myoelectric sensors as input data for the first conversion algorithm. Then, the display unit 303 displays the converted text information (S811).

Additionally, even in the case where the sound information detected by the sound information detection unit 706 includes audio information of the user's speech, if the *S*/*N* of the audio information is poor, the biometric information in addition to the audio information may be used to perform conversion. Alternatively, it may be set to perform conversion only from the biometric information.

### Third Embodiment

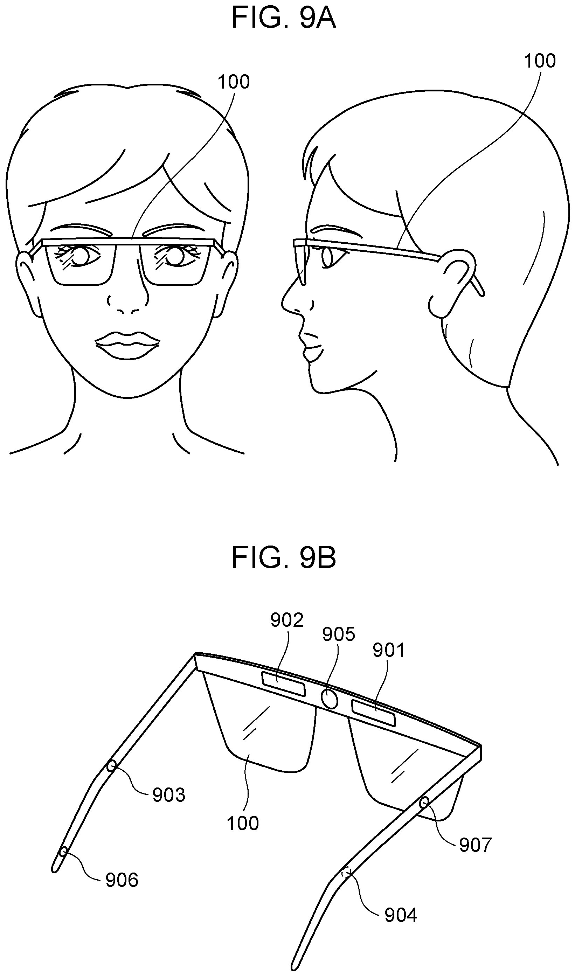

Next, a third embodiment of a text conversion function using the information conversion system of the present invention will be described. Figs. 9A and 9B illustrate schematic diagrams of the detection device 100. The detection device 100 illustrated in Figs. 9A and 9B is applicable to the information conversion system illustrated in Fig. 6.

The detection device 100 is a glasses-type that has each detection unit in a part of the glasses. The detection device 100 is composed of a wearing detection unit 901, a transmitting unit 902, biometric information detection units 903, 904, and 905, and sound information detection units 906 and 907. The biometric information detection units 903 and 904 are myoelectric sensors. The biometric information detection unit 905 is a six-axis acceleration sensor. The myoelectric sensors of the biometric information detection units 903 and 904 are placed on the user's temples. The acceleration sensor of the biometric information detection unit 905 is placed in the user's glabellar region. The sound information detection unit 906 is a bone conduction microphone for detecting sounds generated within the user. The sound information detection unit 907 is a microphone for capturing external environmental sounds.

The wearing detection unit 901, the biometric information detection units 903, 904, and 905, and the sound information detection units 906 and 907 are connected to the transmitting unit 902. The transmitting unit 902 can transmit the wearing detection result detected by the wearing detection unit 901, biometric information detected by the biometric information detection units 903, 904, and 905, and sound information detected by the sound information detection units 906 and 907 to the outside.

The wearing detection unit 901 monitors the outputs from the myoelectric sensors or the acceleration sensor and detects that the user has worn the detection device 100 on the user. The wearing detection unit 901 detects that the user has worn the detection device 100 on the user based on the characteristics of the output waveforms from the myoelectric sensors or acceleration sensor.

Biometric information detected by the biometric information detection units 903, 904, and 905, sound information detected by the sound information detection units 906 and 907, and the wearing detection result detected by the wearing detection unit 901 are transferred by the transmitting unit 902 to the smartphone 300 connected via Bluetooth, for example.

The receiving unit 301 in the application on the smartphone 300 receives data. The determination unit 305 analyzes the sound information received by the receiving unit 301 and determines whether the characteristics of the sound information satisfy a certain condition. The determination unit 305 may make a determination using the wearing detection result detected by the wearing detection unit 901.

The conversion unit 302 converts the biometric information or sound information received by the receiving unit 301 into text information in accordance with the determination result of the determination unit 305. The conversion unit 302 can convert myoelectric information detected by the myoelectric sensors of the biometric information detection units 903 and 904 into text information or audio information. Additionally, the conversion unit 302 can convert acceleration information detected by the acceleration sensor of the biometric information detection unit 905 into text information or audio information. Furthermore, the conversion unit 302 can also convert information including myoelectric information detected by the myoelectric sensors of the biometric information detection units 903 and 904 and acceleration information detected by the acceleration sensor of the biometric information detection unit 905 into text information or audio information.

The text information converted by the conversion unit 302 is displayed on a display, which is the display unit 303 of the smartphone. The sound information output unit 304 plays the audio information converted by the conversion unit 302.

### Fourth Embodiment

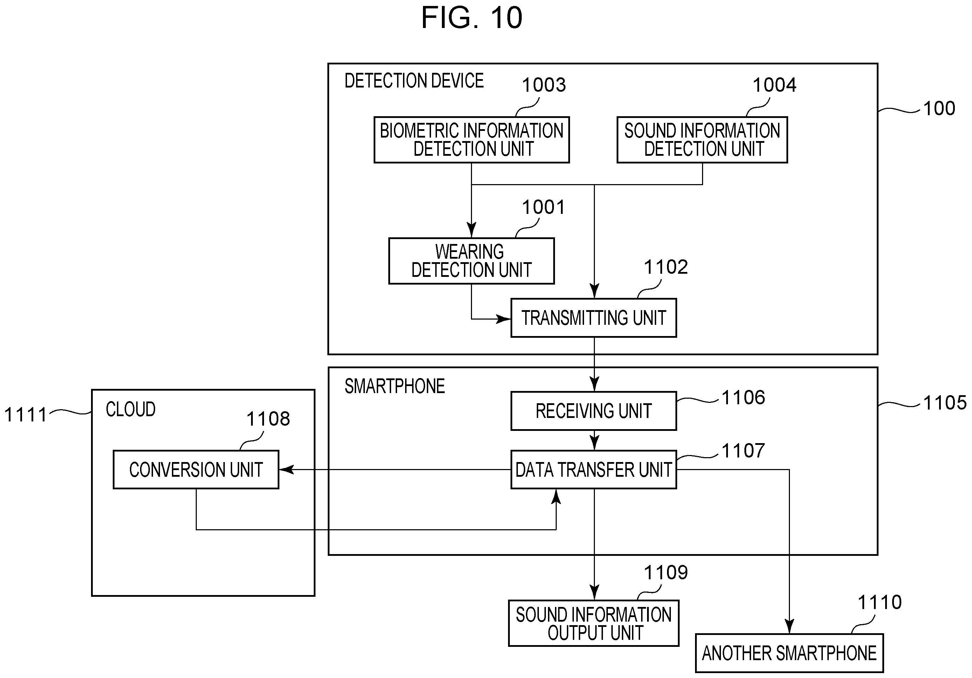

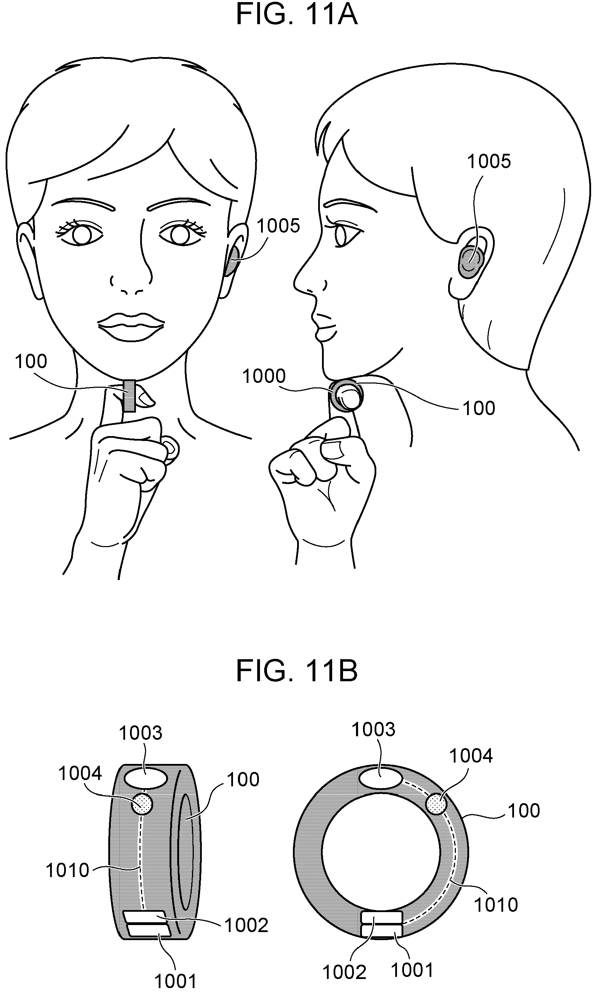

Next, a fourth embodiment of a text conversion function using the information conversion system of the present invention will be described. Fig. 10 illustrates the outline of the information conversion system of the present invention. Figs. 11A and 11B illustrate schematic diagrams of the detection device 100.

The detection device 100 is a ring type (ring shape) that is worn on the user's finger, and can be fitted onto and worn on the user's finger. The detection device 100 is composed of a wearing detection unit 1001, a transmitting unit 1002, a biometric information detection unit 1003, and a sound information detection unit 1004. The biometric information detection unit 1003 is composed of, for example, a six-axis tactile sensor.

The six-axis tactile sensor is a sensor capable of sensing forces in three axial directions and moments in three axial directions. The sound information detection unit 1004 is a microphone that detects sounds such as those generated by the user.

The biometric information detection unit 1003 is installed on the outer side (outer peripheral surface) of the detection device 100, which is a ring type. Therefore, the user can detect biometric information (tactile information) using the biometric information detection unit 1003 by pressing the detection device 100 against the user's skin and bringing the biometric information detection unit 1003 into contact with the user's skin.

The sound information detection unit 1004 is installed on the outer side (outer peripheral surface) of the detection device 100, which is a ring type. Therefore, the sound information detection unit 1004 can detect sounds such as those generated by the user.

The wearing detection unit 1001 monitors the output from the biometric information detection unit 1003 and detects that the user has brought the device into contact with the lower jaw. The wearing detection unit 1001 detects that the detection device 100 has been worn based on the characteristics of the output waveforms from the tactile sensor.

The biometric information detection unit 1003, the sound information detection unit 1004, and the wearing detection unit 1001 are connected to the transmitting unit 1002 via a signal line 1010. The transmitting unit 1002 can transmit biometric information detected by the biometric information detection unit 1003, sound information detected by the sound information detection unit 1004, and the wearing detection result detected by the wearing detection unit 1001 to the outside.

Biometric information detected by the biometric information detection unit 1003 and sound information detected by the sound information detection unit 1004 are transferred wirelessly by the transmitting unit 1002 to a smartphone 1105. A receiving unit 1106 in an application on the smartphone 1105 receives the biometric information detected by the biometric information detection unit 1003 and the sound information detected by the sound information detection unit 1004. A data transfer unit 1107 transfers the biometric information and sound information to a cloud 1111, and a conversion unit 1108 on the cloud 1111 converts the biometric information into audio information. Since the conversion method has been described in the above embodiments, its description will be omitted.

The audio information converted by the conversion unit 1108 is transferred to the data transfer unit 1107. The audio information converted by the conversion unit 1108 is transferred to another smartphone 1110 and played. At the same time, the converted audio is played through a sound information output unit 1109, such as earphones 1005, and the user checks the conversion result.

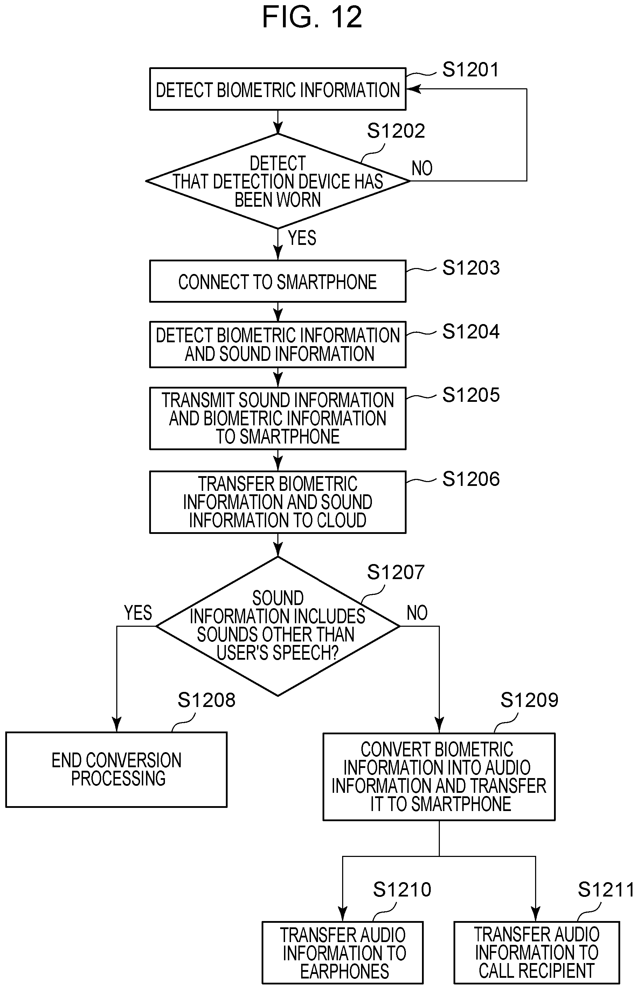

Fig. 12 illustrates a flowchart indicating the operation of the information conversion system of the present invention.

The user wears the detection device 100 at a position where biometric information can be detected by the biometric information detection unit 1003, and sound information can be detected by the sound information detection unit 1004. After the detection device 100 is powered on, the biometric information detection unit 1003 detects biometric information (S1201).

The sampling rate of the tactile sensor is 100 Hz. The wearing detection unit 1001 detects that the detection device 100 has been worn based on the characteristics of the biometric information (characteristics of the output waveforms from the tactile sensor) (S1202).

In response to detection by the wearing detection unit 1001 that the detection device 100 has been worn, the detection device 100 and the smartphone 1110 enter a communicable state. Specifically, the transmitting unit 1002 of the detection device 100 can make a connection to the smartphone 1110 (S1203).

The biometric information detection unit 1003 detects biometric information, and the sound information detection unit 1004 detects sound information (S1204).

The sampling rate of the sound information detection unit 1004 has been set to 16 kHz. The transmitting unit 1002 transmits the sound information and biometric information to the smartphone 1105 (S1205).

The biometric information detected by the biometric information detection unit 1003 and the sound information detected by the sound information detection unit 1004 are transferred from the receiving unit 1106 via the data transfer unit 1107 to the conversion unit 1108 on the cloud 1111 (S1206).

The conversion unit 1108 converts the biometric information into audio information. The conversion unit 1108 determines, from the sound information, sounds related to the user's unintended movements (related to coughing, swallowing saliva, etc.) (S1207).

In the case where the sound information detected by the sound information detection unit 1004 includes sounds related to the user's unintended movements, the process proceeds to S1208; and, in the case where the sound information detected by the sound information detection unit 1004 includes no sounds related to the user's unintended movements, the process proceeds to S1209.

In the case where the sound information detected by the sound information detection unit 1004 includes sounds related to the user's unintended movements, the process ends without converting the biometric information (S1208).

In the case where the sound information detected by the sound information detection unit 1004 includes no sounds related to the user's unintended movements, the conversion unit 1108 converts the biometric information into audio information by using the information obtained from the six-axis tactile sensor as input information for a conversion algorithm. The audio information converted by the conversion unit 1108 is transferred to the smartphone 1105 (S1209).

For the conversion algorithm, a trained model with an architecture configured by a neural network is used. The audio information transferred to the smartphone 1105 is played by the user's earphones 1005 via the data transfer unit 1107 (S1210).

At the same time, the converted audio information is transferred to a call recipient (another smartphone 1110) (S1211).

### Fifth Embodiment

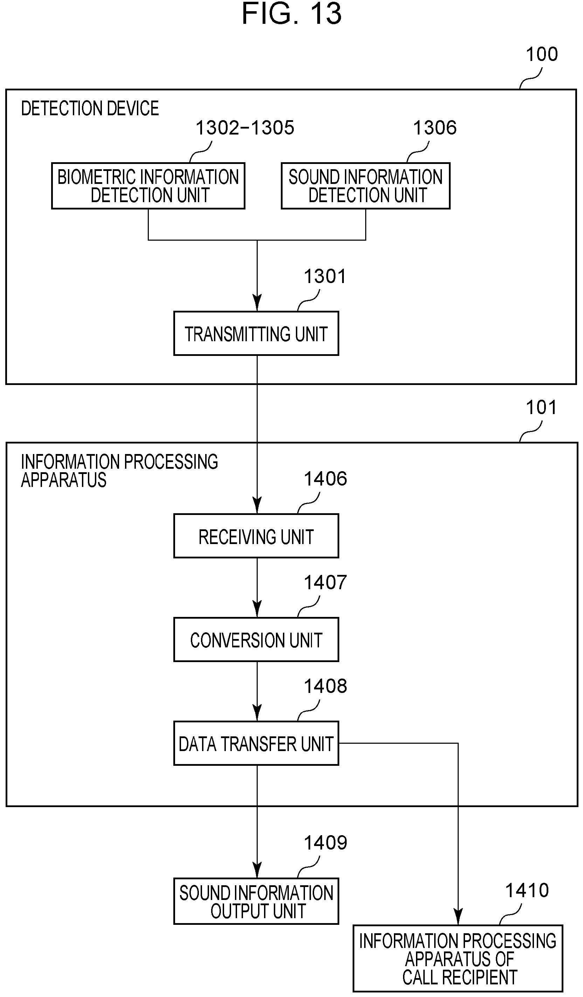

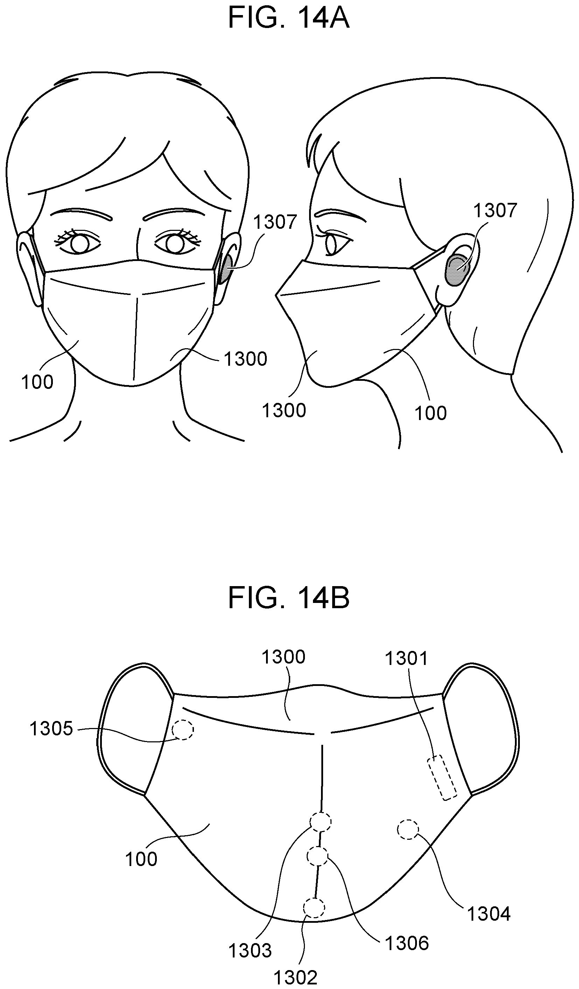

A fifth embodiment of a web conference using the information conversion system of the present invention will be described. Fig. 13 illustrates the outline of the information conversion system of the present invention. Figs. 14A and 14B illustrate schematic diagrams of the detection device 100.

The detection device 100 is a mask-type that has each detection unit in a part of the mask. The detection device 100 is composed of a transmitting unit 1301, biometric information detection units 1302 to 1305, and a sound information detection unit 1306. The biometric information detection units 1302 to 1305 are, for example, six-axis acceleration sensors. The sound information detection unit 1306 is a microphone that detects sounds such as those generated by the user.

The biometric information detection units 1302 to 1305 and the sound information detection unit 1306 are connected to the transmitting unit 1301. The transmitting unit 1301 can transmit biometric information detected by the biometric information detection units 1302 to 1305 and sound information detected by the sound information detection unit 1306 to the outside.

By powering on the detection device 100, the detection device 100 is connected to the information processing apparatus 101. After that, biometric information detected by the biometric information detection units 1302 to 1305 and sound information detected by the sound information detection unit 1306 are transferred wirelessly by the transmitting unit 1301 to the information processing apparatus 101.

A receiving unit 1406 in an application on the information processing apparatus 101 receives biometric information detected by the biometric information detection units 1302 to 1305 and sound information detected by the sound information detection unit 1306. A conversion unit 1407 converts the biometric information into audio information, and the audio information is transferred via a data transfer unit 1408 to an information processing apparatus 1410 of a call recipient (web conference participant). At the same time, the converted audio information is played through a sound information output unit 1409, such as earphones 1307.

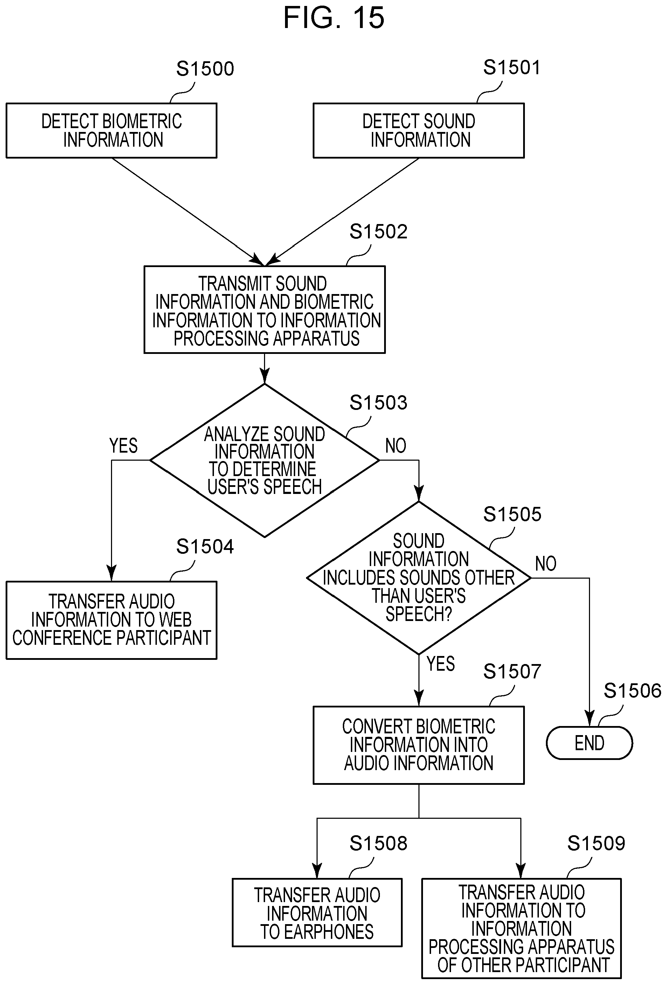

Fig. 15 illustrates a flowchart indicating the operation of the information conversion system of the present invention.

The user wears the detection device 100 at a position where biometric information can be detected by the biometric information detection units 1302 to 1305 and at a position where sound information can be detected by the sound information detection unit 1306. After the detection device 100 is powered on, the biometric information detection units 1302 to 1305 detect biometric information (S1500).

The sampling rate of the acceleration sensor is 1 kHz.

The sound information detection unit 1306 detects sound information. (S1504) The sound information detection unit 1306 is a microphone, and the sampling rate of the sound information detection unit 1306 is 16 kHz. The transmitting unit 1301 transmits the biometric information detected by the biometric information detection units 1302 to 1305 and the sound information detected by the sound information detection unit 1306 to the information processing apparatus 101 (S1502).

The receiving unit 1406 receives the biometric information detected by the biometric information detection units 1302 to 1305 and the sound information detected by the sound information detection unit 1306, and transmits them to the conversion unit 1407. The conversion unit 1407 or a determination unit (not illustrated) determines whether the sound information detected by the sound information detection unit 1306 includes audio information of the user's speech with a model using a neural network (S1503).

In the case where the sound information detected by the sound information detection unit 1306 includes audio information of the user's speech, the process proceeds to S1504; and, in the case where the sound information detected by the sound information detection unit 1306 includes no audio information of the user's speech, the process proceeds to S1505.

In the case where the sound information detected by the sound information detection unit 1306 includes audio information of the user's speech, the audio information is transferred to the information processing apparatus 1410 of the web conference participant (S1504).

In the case where the sound information detected by the sound information detection unit 1306 includes no audio information of the user's speech, it is determined whether the sound information includes sounds related to the user's unintended movements (related to coughing, swallowing saliva, etc.) (S1505).

In the case where the sound information detected by the sound information detection unit 1306 includes sounds related to the user's unintended movements, the process proceeds to S1506; and, in the case where the sound information detected by the sound information detection unit 1306 includes no sounds related to the user's unintended movements, the process proceeds to S1507.

In the case where the sound information detected by the sound information detection unit 1306 includes sounds related to the user's unintended movements, the process ends without converting the biometric information (S1506).

In the case where the sound information detected by the sound information detection unit 1306 includes no sounds related to the user's unintended movements, the conversion unit 1407 converts the biometric information into audio information (S1507).

The audio information is transferred to and played by the user's earphones 1307 via the data transfer unit 1408 (S1508).

At the same time, the converted audio information is transferred to the information processing apparatus 1410 of the web conference participant (S1509).

### Sixth Embodiment

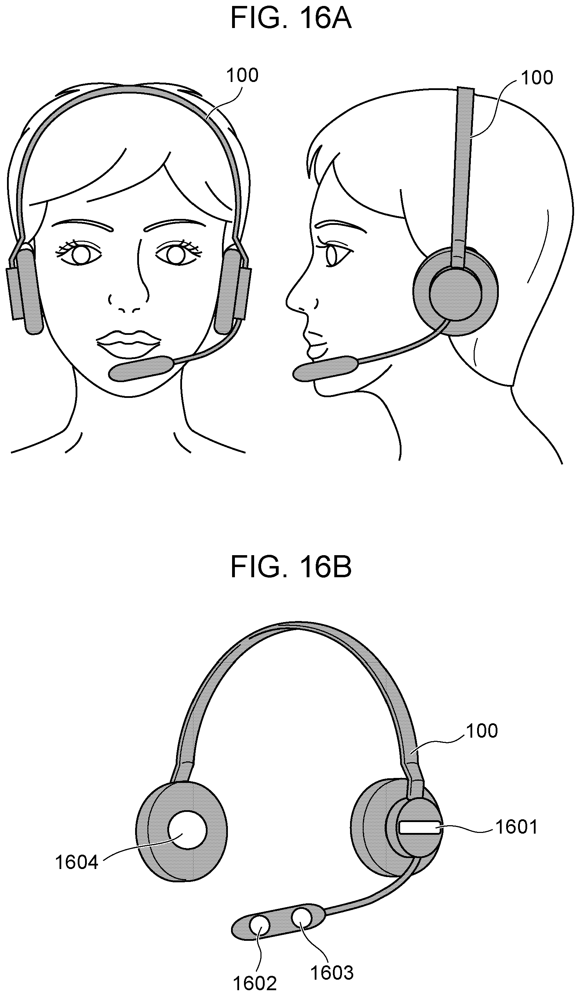

A sixth embodiment of a web conference using the information conversion system of the present invention will be described. Figs. 16A and 16B illustrate schematic diagrams of the detection device 100. The detection device 100 illustrated in Figs. 16A and 16B is applicable to the information conversion system illustrated in Fig. 13.

The detection device 100 is a headset-type that has each detection unit in a part of the headset. The detection device 100 is composed of a transmitting unit 1601, a biometric information detection unit 1602, a sound information detection unit 1603, and earphones 1604. The biometric information detection unit 1602 is an ultrasonic sensor. The ultrasonic sensor is a sensor capable of measuring the distance from an ultrasonic transceiver to an object by transmitting ultrasonic waves in the direction from the ultrasonic transceiver towards the object and receiving the ultrasonic waves reflected from the object. Using the ultrasonic sensor, the biometric information detection unit 1602 can detect ultrasonic information related to changes in the distance from the ultrasonic sensor to the mouth or throat when the user moves the mouth. The sound information detection unit 1603 is a microphone that detects sounds such as those generated by the user.

The biometric information detection unit 1602 and the sound information detection unit 1603 are connected to the transmitting unit 1601. The transmitting unit 1601 can transmit biometric information detected by the biometric information detection unit 1602 and sound information detected by the sound information detection unit 1603 to the outside.

By powering on the detection device 100, the detection device 100 is connected to the information processing apparatus 101. After that, biometric information detected by the biometric information detection unit 1602 and sound information detected by the sound information detection unit 1603 are transferred by the transmitting unit 1601 to the information processing apparatus 101.

The receiving unit 1406 in the application on the information processing apparatus 101 receives biometric information detected by the biometric information detection unit 1602 and sound information detected by the sound information detection unit 1603. The conversion unit 1407 converts the biometric information into audio information, and the audio information is transferred via the data transfer unit 1408 to the information processing apparatus 1410 of the call recipient (web conference participant). At the same time, the converted audio information is played through the sound information output unit 1409, such as the earphones 1604.

The present invention can also be realized by a process of supplying a program that implements one or more functions of the above-described embodiments to a system or apparatus via a network or storage medium, and having one or more processors in the system or apparatus read and execute the program. Additionally, the present invention can also be realized by a circuit (e.g., ASIC) that implements one or more functions. The program and a computer-readable storage medium on which the program is stored are included in the present invention.

Note that the embodiments of the present invention described above are merely examples of specific implementations of the present invention, and the technical scope of the present invention should not be interpreted as limited by these embodiments. That is, the present invention can be implemented in various forms without departing from its technical concept or main features.

This application claims priority from Japanese Patent Application No. 2022-064525, filed April 8, 2022, which is hereby incorporated by reference herein in its entirety.

## Claims

1. An information conversion system comprising:
a biometric information detection unit that detects biometric information from one or more parts of a user;
a sound information detection unit that detects sound information;
a determination unit that determines whether a characteristic of the sound information satisfies a certain condition; and
a conversion unit that outputs text information or audio information converted from the biometric information detected by the biometric information detection unit using a first conversion method in a case where the characteristic of the sound information detected by the sound information detection unit does not satisfy the certain condition, and
outputs text information converted from the sound information detected by the sound information detection unit using a second conversion method in a case where the characteristic of the sound information satisfies the certain condition.

2. The information conversion system according to Claim **1,** wherein, in a case where the characteristic of the sound information satisfies the certain condition, the conversion unit outputs text information or audio information converted from information including the sound information detected by the sound information detection unit and the biometric information detected by the biometric information detection unit, using a third conversion method.

3. The information conversion system according to Claim 1, wherein the conversion unit switches from the first conversion method to the second conversion method or from the second conversion method to the first conversion method depending on the characteristic of the sound information detected by the sound information detection unit, and outputs text information or audio information converted by the switched conversion method.

4. The information conversion system according to Claim 1, wherein the sound information detection unit is composed of at least two or more detection units.

5. The information conversion system according to Claim 4, wherein the sound information detection unit includes a sound information detection unit detecting sound information generated within a body and a sound information detection unit detecting external sound.

6. The information conversion system according to Claim 5, wherein the sound information detection unit detecting sound information generated within the body is a bone conduction microphone or a skin conduction microphone.

7. The information conversion system according to Claim 1, wherein, in a case where the determination unit determines that the sound information detected by the sound information detection unit contains noise, the conversion unit does not convert the biometric information into either text information or audio information.

8. The information conversion system according to Claim 1, wherein, in a case where the determination unit determines that a volume of sound related to a user's speech in the sound information detected by the sound information detection unit is greater than a threshold, the conversion unit converts the sound information into text information or audio information.

9. The information conversion system according to Claim 1, wherein, in a case where the determination unit determines that an S/N of sound related to a user's speech in the sound information detected by the sound information detection unit is greater than a threshold, the conversion unit converts the sound information into text information or audio information.

10. The information conversion system according to Claim 1, wherein, in a case where the determination unit determines that a volume of sound related to a user's speech in the sound information detected by the sound information detection unit is less than or equal to a threshold, the conversion unit converts the biometric information into text information or audio information.

11. The information conversion system according to Claim 1, wherein, in a case where the determination unit determines that the sound information detected by the sound information detection unit corresponds to a user's speech period, the conversion unit converts the sound information into text information or audio information.

12. The information conversion system according to Claim 1, wherein, in a case where the determination unit determines that the sound information detected by the sound information detection unit corresponds to a user's non-speech period, the conversion unit converts the biometric information into text information or audio information.

13. The information conversion system according to Claim 1, wherein, depending on the characteristic of the sound information detected by the sound information detection unit, the conversion unit sets either the first conversion method of converting biometric information into text information or audio information or the second conversion method of converting the sound information into text information, and converts the biometric information or the sound information into text information or audio information.

14. The information conversion system according to Claim 1, wherein the biometric information includes at least one of myoelectric information, acceleration information, ultrasonic information, tactile information, light information, and pressure information.

15. The information conversion system according to Claim 1, wherein the conversion unit further converts text information converted from the biometric information into audio information.

16. An information conversion system comprising:
a biometric information detection unit that detects biometric information from one or more parts of a user;
a sound information detection unit that detects sound information;
a determination unit that determines whether a characteristic of the sound information satisfies a certain condition; and
a conversion unit that outputs text information or audio information converted from the biometric information detected by the biometric information detection unit using a first conversion method in a case where the characteristic of the sound information detected by the sound information detection unit does not satisfy the certain condition, and
outputs text information converted from information including the sound information and the biometric information using a third conversion method in a case where the characteristic of the sound information satisfies the certain condition.

17. The information conversion system according to Claim 16, wherein the conversion unit switches from the first conversion method to the third conversion method or from the third conversion method to the first conversion method depending on the characteristic of the sound information detected by the sound information detection unit, and outputs text information or audio information converted by the switched conversion method.

18. The information conversion system according to Claim 1, comprising:
a wearing detection unit that detects that the biometric information detection unit has been worn on the user,
wherein, in response to detection by the wearing detection unit that the biometric information detection unit has been worn on the user, the conversion unit becomes able to convert the biometric information detected by the biometric information detection unit into text information or audio information.

19. The information conversion system according to Claim 18, wherein the wearing detection unit detects that the biometric information detection unit has been worn on the user by analyzing the biometric information detected by the biometric information detection unit.

20. The information conversion system according to Claim 1, comprising:
a detection device including the biometric information detection unit, the sound information detection unit, and a transmitting unit that transmits the biometric information detected by the biometric information detection unit and the sound information detected by the sound information detection unit; and
an information processing apparatus including a receiving unit that receives the biometric information and audio information transmitted from the transmitting unit, the determination unit, and the conversion unit.

21. The information conversion system according to Claim 20, comprising:
a wearing detection unit that detects that the biometric information detection unit has been worn on the user,
wherein, in response to detection by the wearing detection unit that the biometric information detection unit has been worn on the user, the detection device and the information processing apparatus enter a communicable state.

22. The information conversion system according to Claim 20, wherein the detection device is one of a choker type, a jaw mask type, a glasses type, a ring type, a mask type, and a headset type.

23. The information conversion system according to Claim 1, comprising a processing unit that evaluates the biometric information detected by the biometric information detection unit based on a certain evaluation criterion, and deletes text information or audio information corresponding to the biometric information not satisfying the certain evaluation criterion.

24. The information conversion system according to Claim 1, wherein, in a case where the characteristic of the sound information detected by the sound information detection unit does not satisfy the certain condition, the conversion unit converts the biometric information detected by the biometric information detection unit into text information or audio information using the first conversion method, and, in a case where the characteristic of the sound information satisfies the certain condition, the conversion unit converts the sound information into text information using the second conversion method.

25. The information conversion system according to Claim 1, wherein a trained model to be used in the first conversion method is updated based on correspondence between text information converted by the second conversion method using the sound information detected by the sound information detection unit and the biometric information obtained by the biometric information detection unit obtained at a same time as the sound information detected by the sound information detection unit.

26. An information processing apparatus comprising:
a receiving unit that receives biometric information detected by a biometric information detection unit and sound information detected by a sound information detection unit;
a determination unit that determines whether a characteristic of the sound information satisfies a certain condition; and
a conversion unit that outputs text information or audio information converted from the biometric information detected by the biometric information detection unit using a first conversion method in a case where the characteristic of the sound information detected by the sound information detection unit does not satisfy the certain condition, and outputs text information converted from the sound information using a second conversion method in a case where the characteristic of the sound information satisfies the certain condition.

27. An information processing method comprising:
a step of receiving biometric information detected by a biometric information detection unit and sound information detected by a sound information detection unit;
a step of determining whether a characteristic of the sound information satisfies a certain condition; and
a step of outputting text information or audio information converted from the biometric information detected by the biometric information detection unit using a first conversion method in a case where the characteristic of the sound information detected by the sound information detection unit does not satisfy the certain condition, and outputting text information converted from the sound information using a second conversion method in a case where the characteristic of the sound information satisfies the certain condition.

28. A program for causing a computer to execute the information processing method according to Claim 27.
